(19) Europäisches Patentamt
European Patent Office
Office européen des brevets

(11) Publication number: **0 045 790**
**B1**

(12) **EUROPEAN PATENT SPECIFICATION**

(45) Date of publication of patent specification: **30.10.85**

(51) Int. Cl.$^4$: **A 61 F 5/46**

(21) Application number: **81900618.0**

(22) Date of filing: **18.02.81**

(86) International application number: **PCT/US81/00205**

(87) International publication number: **WO 81/02389 03.09.81 Gazette 81/21**

(54) **CONFORMING VALVED CERVICAL CAP AND MOLDING METHOD.**

(30) Priority: **19.02.80 US 122541**
**18.11.80 US 207904**

(43) Date of publication of application:
**17.02.82 Bulletin 82/07**

(45) Publication of the grant of the patent:
**30.10.85 Bulletin 85/44**

(84) Designated Contracting States:
**DE FR GB**

(56) References cited:
**US-A-2 580 133**
**US-A-3 216 422**
**US-A-3 786 807**
**US-A-3 952 737**
**US-A-4 007 249**
**US-A-4 198 965**

(73) Proprietor: **UNIVERSITY PATENTS, INC.**
**537 Newtown Avenue**
**Norwalk Connecticut 06851 (US)**
(73) Proprietor: **CONTRACAP, INC.**
**999 Plaza Drive**
**Schaumburg, Illinois 60195 (US)**

(72) Inventor: **Freese, Uwe E.**
**238 North Forest**
**Oak Park, IL 60302 (US)**
Inventor: **Goepp, Robert A.**
**5928 North Kilbourn**
**Chicago, IL 60646 (US)**
Inventor: **Loeb, Marvin P.**
**7350 North Washtenaw Avenue**
**Chicago, IL 60645 (US)**

(74) Representative: **Heath, Derek James et al**
**Bromhead & Co. 30 Cursitor Street Chancery Lane**
**London EC4A 1LT (GB)**

**EP 0 045 790 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Technical field

This invention relates to contraceptive devices, and more particularly, to a cervical cap and method of molding the same to conform to the shape of the cervix uteri.

Background of the invention

Caps for cervix uteri as birth control devices are well known and have been found among the artifacts of antiquity. It has been reported that Aetius of Amida suggested the use of the skin of a pomegranate cut into a hollow cup and that beeswax discs fashioned to fit over the cervix have been used in Europe.

Conventional cervical caps are made in many sizes to accommodate the various cervix sizes normally encountered, however, with such caps the fit for a particular individual is usually inexact and probably a compromise. Usually, the cervix is either wedged into the cap or a compressive retainer or clamp is utilized. As a result, conventional caps often become dislodged during normal body movement, and particularly during coitus, and have to be removed periodically to accommodate normal uterine discharges.

Typifying the many types of cervical caps that have been developed are those shown in U.S. Patents Nos. 2,836,177, 3,952,737 and 4,007,249, and German Patent No. 475,496 as well as in *Das Weibliche Gebar-Unvermogen* by F. A. Wilde in Berlin in 1838 according to U.S. Patent No. 4,007,249. These cervical caps have met with varying degrees of success. In particular, U.S. Patent No. 3,952,737 shows a cap with a flexible shell having a concave inner surface which receives a portion of the cervix uteri. The cap shown in U.S. Patent No. 4,007,249 is made while the wall of the vagina is expanded by means of a speculum. Such an expedient tends to deform the cervix and thus precludes a good fit between the cap and the cervix. Additionally, medical grade silicone rubber, the material of choice in said patent for making the cap is not readily wettable, thus surface tension cannot be relied upon to hold the cap properly in place on the cervix. In addition, some medical grade silicone rubbers are porous, and cervical mucous, uterine discharges and bacteria are likely to accumulate in the pores, thereby causing unpleasant odor, in addition to a risk of infection. In such instances, cervical caps may be usable only for limited time period, e.g., about 6—8 hours.

Summary of the invention

The present invention seeks to solve the problem of dislodgement during intercourse or customary physical activities. Accordingly, the present invention is directed to a cervical cap with a flexible shell having a concave inner surface of sufficient depth to receive a major portion, but not all of a cervix uteri, characterized by a form-assuming internal liner covering the concave inner surface defining a wettable, matingly fitting and complementing contact surface for the exocervical surface of the cervix uteri.

Such a cap may thus be provided with an inner wettable liner that is impermeable or non-porous, and which conforms to the cervix uteri of the female for whom the cap is made, so that the surface tension created by a layer of mucous between the inner wettable surface of the cap and the exterior of the cervix will hold the cap in place and prevent such dislodgement.

An improved cervical cap can thus be provided which is comfortable, durable, reliable and effective. The novel cervical cap remains in place during vigorous sexual intercourse as well as during normal body movement, since it conforms to the surface of the cervix and enjoys the holding force of surface tension. The cap can be comfortably worn for extended periods of time, such as for months or even years, without removal.

The cervical cap can be provided with a one-way valving means which permits egress of uterine discharges but prevents ingress of sperm. The cap may include a prefabricated non-porous shell provided with a soft resilient elastomeric lining which is secured to the shell and defines a wettable, non-porous contact surface to fit and resiliently complement the exocervical surface of the cervix uteri while accommodating the usual cyclical configuration changes of the cervix. The valving means may include an aperture at about the apex of the shell and a flexible web overlying the aperture and coacting with the shell.

A cervical cap made in accordance with the present invention may be passive in that it exerts no pressure on the cervix when in place. Such a cap, while in place, is capable of limited sliding movement over the surface of the cervix and exerts no localized pressure on the cervix, thereby avoiding hypertrophy, hyperplasia, dyskeratotic changes in the squamous epithelium, and the like.

In one embodiment the cap is custom fitted. To custom-fit and contour the cervical cap, an elastomeric layer is molded and cured in situ adjacent the exocervical surface of the cervix uteri of the wearer. A liner or plug covers the valve during molding and curing to prevent the elastomeric layer from clogging or otherwise impairing operation of the valve. The plug can be a T-plug, a bridge plug or an umbrella plug which is preferably removed from the valve after the elastomeric layer has cured. In the preferred method, the flexible outer liner sheath separates the inner wettable elastomeric layer from the exocervical surface during the in situ molding and curing process. After the elastomeric layer has cured, the liner sheath is trimmed or removed.

In one form, a removable outer liner sheath is used to separate the cervix from an inner wettable elastomeric material during the molding and curing process. In another form, the inner elastomeric material is non-wettable and is permanently covered by a wettable outer liner sheath which is substantially

contiguous with the exocervical surface of the cervix uteri to obtain the desired surface tension effect. In both instances, the defined contact surface is non-porous.

In another preferred embodiment of the present invention, the cap comprises a generally dome-like flexible shell and a resilient, form-assuming internal liner. The flexible shell is of sufficient depth to receive therein a major portion but not all of the cervix uteri. To maximize the effect of the mucous surface tension the area of intimate contact between the cap and the cervix uteri should be maximized and to this end it is preferable to maximize the available surface of the form-assuming internal liner. The liner may extend beyond the shell to further increase the available contact area. The liner is readily deformable upon contact with the cervix uteri without substantial deformation of contiguous portion of the cervix uteri, i.e. the liner has a lower hardness value (is softer) than the body tissue in contact therewith when the cap is in place. The cervical caps embodying the present invention can be fabricated in various sizes having diameter and depth dimensions sufficient to accommodate the usual uterine size variations.

A more detailed explanation of the invention is provided in the following description and appended claims taken in conjunction with the accompanying drawings.

## Brief description of the drawings

Figure 1 is a cross-sectional view of a cervical cap being molded and cured in situ contiguous with the exocervical surface of the cervix uteri and with an outer liner sheath which can be removed or trimmed in accordance with principles of the present invention;

Figure 2 is an enlarged cross-sectional view of a cervical cap with a trimmed wettable liner sheath embodying the present invention;

Figure 3 is a fragmentary cross-sectional view of a portion of another cervical cap in accordance with the present invention;

Figure 4 is a fragmentary cross-sectional view of a portion of still another cervical cap in accordance with the present invention;

Figure 5 is a fragmentary view of a portion of a further cervical cap manufactured in accordance with the present invention but before final trimming;

Figure 6 is a fragmentary cross-sectional view of a portion of a cervical cap which has been fitted with a canal button or plug and liner sheath for in situ molding and curing in accordance with the present invention;

Figure 7 is a fragmentary cross-sectional view of a portion of a cervical cap fitted with another plug and liner sheath for in situ molding and curing in accordance with the present invention;

Figure 8 is a fragmentary cross-sectional view of a portion of a cervical cap fitted with a bridge plug and liner sheath for in situ molding and curing in accordance with the present invention;

Figure 9 is a fragmentary cross-sectional view of a portion of a cervical cap fitted with a bridge plug for in situ molding and curing in accordance with the present invention;

Figure 10 is a fragmentary cross-sectional view of a portion of a cervical cap utilizing a T-plug for in situ molding and curing in accordance with the present invention;

Figure 11 is a perspective view of a cervical cap having a slit rim with independently movable or deformable rim sections in accordance with the present invention;

Figure 12 is a cross-sectional view of a cervical cap having a deformable liner contiguous with the exocervical surface of the cervix uteri;

Figure 13 is an enlarged cross-sectional view of another valved cervical cap embodying the present invention;

Figure 14 is an enlarged perspective view of a valved cervical cap embodying the present invention;

Figure 15 is a fragmentary cross-sectional view of a valved cervical cap embodying the present invention and illustrating yet another one-way valve construction; and

Figures 16 and 17 are cross-sectional elevational views illustrating a device suitable for manufacturing a cervical cap embodying the present invention and the operation thereof.

## Detailed description of the preferred embodiments

Referring to Figure 1 of the drawings, a custom-fit cervical cap 10 is shown being molded and cured in situ on the exocervical surface 12 of the cervix uteri 14, i.e., on the portio vaginalis cervicis or that portion of the uterus 16 that protrudes into the vagina 18, of the woman who is to wear the cervical cap assembly and for whom the cervical cap assembly is being custom fitted and contoured. The uterus 16 is supported by broad ligaments 20 and 21 and defines fundus that terminates in the cervical os 24. The vaginal wall 25 together with cervix 14 defines the fornices vaginae, i.e., the lateral fornices 26 and 27 as well as the anterior and posterior fornices (not shown).

The cervical cap 10 provides a non-invasive birth control device which is effective and reliable to substantially deter impregnation. Cap 10 includes a flexible and deformable cervical shell 30 which is preformed. Shell 30 is non-porous and can be dome-like and having a slightly flattened apex, or cup-shaped, with a concave inner surface 32 and a central valve opening or aperture 40 at its apex.

Shell 30 is operatively connected to a one-way valving means 34 which defines a tunnel or channel that permits unidirectional flow in the direction away from the cervical os 24 but not in the reverse direction. Valving means 34 opens under minimal predetermined positive pressure, which may be 5 millimeters of

mercury or less, to permit the outflow of uterine discharges, such as menstrual flow, mucus, and the like, without disturbing the positioning of the cervical cap, but prevents the entry of sperm therethrough into fundus 22. Cervical cap 10 is of a sufficient depth to receive a major portion but not all of the cervix uteri 14.

A soft, pliable intermediate resilient elastomeric contact layer or cushion 36 of a wettable, non-porous material is moldably or adhesively bonded or otherwise attached to the concave inner surface 32 of shell 30. Elastomeric layer 36 has a custom-fit contoured surface 38 which is permanently shaped and contoured to matingly fit and resiliently complement the exocervical surface 12 of the cervix uteri 14. In the preferred embodiment, elastomeric layer 36 extends from a position adjacent valve opening 40 (Figure 2) to a position adjacent margin or rim 42 of shell 30. Elastomeric layer 36 is sufficiently flexible, compressible and form-assuming to accommodate the usual cyclical configuration changes that the uterus and the cervix undergo.

As best shown in Figure 2, a removable protective flexible sheet-like liner or liner sheath 44 isolates and separates elastomeric layer 36 from the exocervical surface 12 (Figure 1) of the cervix uteri 14 during molding and curing. Liner sheath 44 is moldably or adhesively bonded or otherwise connected to the concave inner surface 32 of shell 30 adjacent valve opening 40 and extends to and is connected against the inner edge of rim 42 so as to completely cover elastomeric layer 36. Liner sheath 44 has valve covering portions 44a which protectively covers valve opening 40 and valve 34 during molding and curing. Liner sheath can also extend over rim 42 so as to form a graspable collar 44b. Liner sheath 44 is made of a flexible, medical-grade, liquid-impervious material which is durable and resistant to tearing. After molding and curing, liner sheath 44, including section 44a thereof, is removed. Cap 10 fits onto the cervix uteri 14 and is slidably held in place by the surface tension of mucous discharge that flows generally continuously from the cervical os 24 along the exocervical surface 12 of the cervix uteri 14 and acts on wettable elastomeric layer 36. In this manner, removeable liner sheath 44, provides a cervix uteri-engaging means that does not exert localized pressure on the cervical surface in contact therewith. In the preferred embodiment, the combination of wettable elastomeric layer 36 and shell 30 exhibits a resiliency that does not deform or distort the cervix uteri 14. Preferably layer 36 and shell 30 are made of the same liquid impervious, non-porous material or of moldably bondable compatible materials.

In some circumstances it may be desirable to use a permanent, wettable, non-porous liner sheath which covers and is moldably bonded, adhesively connected or otherwise permanently secured to a non-wettable and/or porous elastomeric layer. In such cases the elastomeric lining of the shell is constituted by the elastomeric layer as well as the liner sheath. Care should be taken, however, to remove valve covering portions 44a or to sever the valve covering portion 44a from liner sheath 44 about valve opening 40 to assure operability of valving means 34. In such circumstances, the outer liner sheath 44 and intermediate elastomeric layer 36 together provide a multi-layer, resilient elastomeric lining having a non-porous and wettable contact surface.

The preferred method to make, custom fit and contour the cervical cap 10 to the exocervical surface 12 of the cervix uteri 14 is to completely cover valving means 34 and valve opening 40 with portions 44a (Figure 1) of liner sheath 44 and to bond or otherwise attach the peripheral part of those valve covering portions 44a to the concave inner surface 32 of shell 30 about valve opening 40. For added securement, valve covering portions 44a can also be bonded or otherwise attached to valving means 34.

Liner sheath 44 is positioned along the concave inner surface 32 of preformed shell 30 so that its unattached portions 44b are spaced inwardly of the cap's rim 42 to form an elastomeric material-receiving pocket, opening, spacing or cavity between the concave inner surface 32 of shell 30 and liner sheath 44. The pocket is then filled with a wettable paste-like elastomeric-forming material which is moldable and curable at about body temperature to form elastomeric layer 36.

After the pocket has been filled with elastomeric material, to which the catalyst or curing agent has been added, the free end or distal end 44b (Figure 1) of liner sheath 44 is turned over the edge of rim 42 and may be bonded or otherwise attached to rim 42 or left free. In order to insert the cap in the vagina, the wall of the vagina is initially expanded with vagina speculum blades to facilitate access and to expose the exocervical surface 12 of the cervix uteri 14. The cap is then inserted in the vagina until the liner sheath 44 is firmly positioned against the exocervical surface 12 of the cervix uteri 14. Liner sheath 44 prevents the paste-like elastomeric-forming material from physically contacting the exocervical surface 12 of the cervix uteri 14.

The speculum, when in position, pulls on the ligaments supporting the uterus and cervix and thus distorts the cervix uteri. Therefore, it is important to remove the speculum from the vagina or to close the speculum blades so as to permit the cervix to return to its normal, undistorted configuration before the paste-like material has taken a permanent set.

The paste-like material is molded and cured in situ adjacent the exocervical surface, on the pocket side of liner sheath 44, to form wettable elastomeric layer 36 which generally conforms to, matingly fits and resiliently complements the natural, undistorted exocervical surface 12 of the cervix uteri 14. The curing temperature can be slightly above the body temperature so long as the exocervical contacting surface 46 of liner 44 is at a comfortable temperature to the person being custom fitted with the cervical cap 10 during in situ molding and curing.

After layer 36 has cured or set, the term "set" being used herein interchangeably with the term "cured", cap 10 is removed from the vagina and the excess portions of liner 44 extending over rim 42 are

trimmed or, in the case of liner 44 extending over rim 32 to form the graspable collar 44b, said liner 44 is detached. The valve aperture-covering portions 44a of liner 44 are thereby removed to provide a cap 10 as shown in Figure 2.

Liner sheath 44 and shell 30 can be adhesively connected to elastomeric layer 36 before molding or after curing if they are not connectable thereto during the molding process. Alternatively, liner 44 can be directly bonded or otherwise attached to the inner surface of shell 30 to securely entrap, enclose and wedge the unattached elastomeric layer 36 between the liner sheath and the concave inner surface 32 of shell 30 without exposing the seam of the bond. Shell 30 is preformed in various sizes so that a shell size providing the desired thickness of layer 36 can be selected for a given user. The valving means can be prefabricated with the shell. Of course, removal of liner sheath 44 necessitates that elastomeric layer 36 be liquid impermeable when cured. Any minor surface imperfections in layer 36 that are noticeable after liner sheath 44 is removed can be filled in, using the same curable material that is used for forming layer 36 initially or in any other convenient manner.

While the above cervical cap 10 is preferred, in some circumstances it may be desirable to use a non-wettable and/or porous elastomeric material to form layer 36 and to permanently retain wettable, non-porous liner 44 after the elastomeric layer 36 has been cured. In such a case a wettable, non-porous outer liner sheath material and a non-wettable elastomeric material may be used together as indicated above. Because of the noval custom fit and contoured method of in situ molding and curing described above, liner 44 sheath is composed of a medical grade, liquid-impervious, non-porous material which is generally unabrasive and non-irritating to the wearer, directly contacting the exocervical surface of the cervix uteri.

Alternatively, use of liner sheath 44 can be confined to covering valving means 34 and valve opening 40. The wettable, non-porous elastomeric material 36 can be activated by mixing with an activating agent or catalyst, and an ample amount of same is spread around the inner surface 32 of shell 30, after which the cap 10 is inserted onto the cervix during the curing process. After curing, cap 10 is removed, liner sheath 44 is detached and any excess portion of layer 36 is removed by trimming. The cap is then reinserted.

The cervical caps of Figures 3—11 are substantially similar to the cervical cap of Figures 1 and 2 except as explained below. For ease of understanding and for clarity, similar parts and components of the cervical caps of Figures 3—10 have been given part numbers with the same last two digits as similar parts and components of the cervical cap of Figures 1 and 2.

In the embodiment shown in Figure 3, the distal end 145 of liner sheath 144 is bent over the rim or skirt 142 of shell 130 and bonded otherwise secured to rim 142 after the elastomeric layer 136 has cured and the cap removed from the vagina. The excess portions of liner sheath 144 are then trimmed.

In the embodiment of Figure 4, liner sheath 244 is bonded or otherwise secured to the inside edge of rim 242. The resultant cured layer 236 has a narrow beveled and wedge shape of reduced thickness adjacent rim 242 as compared to a more rounded shape shown in the previous embodiments.

In the embodiment shown in Figure 5, liner sheath 344 extends beyond rim 342 and is reverse bent, forming a pocket, to be attached by adhesive or otherwise bonded to the rim 342 of exterior of shell 330. The cervical cap 310 is inserted onto the cervix and molded and cured in situ as explained above. After the moldable material forming layer 336 has been cured, the cap 310 is removed and the excess elastomeric material 336 as well as excess liner margin 348 are cut and removed along the severance line C to form an inwardly converging tapered surface or end portion 347 having a generally frusto-conical configuration, which extends beyond shell 330 and can be cut with a series of slits to form deformable sections, as hereinafter described. Any remaining portion of liner sheath 44b can be detached. As a result, the peripheral marginal portion or rim of cap 310 is thinner than the average wall thickness of the cap and is beveled toward the fornices when the cap is in place.

In the embodiment of Figure 5, shell 330 has a tapered body 348, which is beveled or tapered towards rim 342, with a maximum thickness of about 0.2 millimeters at rim 342. Other thicknesses can be used. If desired, the cervical caps of the other embodiments of this invention can also have a tapered body.

In the embodiment of Figure 6, a canal button or plug 450 is used in lieu of valve aperture-covering liner portions 44a (Figure 1). Plug 450 is positioned in the valve opening 440 and bonded or otherwise removably secured to valve means 434 before the moldable elastomeric-forming material is poured into the pocket between the concave inner surface 432 of shell 430 and liner sheath 444. Plug 450 is substantially solid and covers valve means 434. After plug 450 is attached to valve means 434, liner sheath 444 is adhesively connected to plug 450, if it is not integrally connected thereto, before the moldable material is poured into the pocket. After the moldable material has been poured into the pocket, the free end of liner sheath 444 is bent over rim 42 and the cap is positioned over the cervix and molded and cured in situ as described with respect to Figure 1. When the moldable material has cured, the cap is removed from the cervix, the excess liner extending over rim is trimmed and secured to rim, if it is not already moldably bonded to rim, and plug 450 is removed.

The method shown in the embodiment of Figure 7 is substantially similar to the embodiment of Figure 6, except that canal button or plug 552 is separated from and not connected to liner sheath 544.

The method shown in the embodiment of Figure 8 is substantially similar to the embodiment of Figure 6 except that a bridge plug or umbrella plug 654 is used in lieu of canal button or plug 450 (Figure 6). Bridge plug 654 bridges the valve opening 640 and is adhesively attached to the concave inner surface 632 of shell

630 adjacent the valve opening 640. Liner sheath 644 is adhesively attached to bridge plug 654 if not already integrally connected thereto.

The method shown in the embodiment of Figure 9 is similar to that illustrated in Figure 8, except that no liner sheath is used and the moldable material forming layer 736 directly contacts the exocervical surface of the cervix uteri when the cap assembly is molded and cured in situ.

The method shown in embodiment of Figure 10 is similar to the embodiment of Figure 9, except that an inverted T-shaped plug or T-plug 856 is used in lieu of bridge plug 754 (Figure 9) to give stability for the alignment of the internal opening or port of a cap tunnel with the cervical os. T-plug 856 includes base 858 which bridges valve opening 840 and rounded stem 861 having concave sides 860. Stem 861 is contoured to be received into the cervical os 824 and retained in position during molding and curing. The periphery of base 858 is contoured to provide a complementary snap fit engagement with the inner edge of shell 830 defining the valve opening or aperture 840.

Alternatively, a temporary liner sheath can be provided connected to the T-plug 856 of Figure 10. In such a case the liner sheath as well as plug 856 are removed when the material forming elastomeric layer 836 has been cured.

Plugs and liner sheaths having different shapes and thicknesses can be used without departing from the scope of this invention. Furthermore, it may be desirable in some circumstances, to remove part or all of the liner sheaths 44 in the embodiments above utilizing liner sheaths after the elastomeric-forming material 36 has been cured and the cap assembly removed from the cervix uteri.

The plugs are, of course, sterile when used, and preferably have a configuration approximating that of a dumbell with one end somewhat flattened, as illustrated in Figure 10 for T-plug 856. Preferably the overall length of the plug is about 15 millimeters and the end of the plug entering the cervical os is about 7 millimeters in diameter.

As shown in Figure 11, the rim or skirt 66 of the cap assembly 63 can also be slit, such as at equally spaced circumferential intervals about rim 66 to provide arcuate longitudinal slits 62, i.e., slits along the great circles of longitude of hemispherical shell 68, which form separate bendable rim sections 64. Rim sections 64 assist in preventing the cervical cap assembly from becoming dislodged during vigorous sexual intercourse and strenuous exercises. In some circumstances it may be desirable to attach a separate deformable skirt to the perimeter or rim of the shell. In addition, shell 68 can be tapered or reduced in thickness by mechanical means, to a dimension of about 0.2 mm., after curing and removal, prior to mechanically creating slits 62.

An alternative series of embodiments utilizes a deformable liner which is preformed and shapes itself to the cervix uteri when worn. The embodiments utilizing a deformable liner and device for their manufacture are shown in Figures 12—17 and will be designated by the 900 series. Referring to Figure 12, a valve cervical cap 910 having a deformable liner is shown positioned on exocervical surface 912 of cervix uteri 914, i.e., on the portio vaginalis cervicis or that portion of uterus 916 that protrudes into vagina 918. Uterus 916 is supported by broad ligaments 920 and 921, and defines fundus 922 that terminates in cervical os 924. Vaginal wall 925 together with cervix 914 define the fornices vaginae, i.e., lateral fornices 926 and 927 as well as the anterior and posterior fornices (not shown).

Cervical cap 910 includes a cup-shaped or dome-like shell 930, fabricated of a flexible material such as rubber, synthetic rubber, an elastomeric synthetic polymer, or the like, and a resilient foam lining 932 about the periphery of the inner concave surface of shell 930. Foam lining 932 can cover all or a major portion of the inner surface of shell 930. Foam lining 932 can terminate at about the rim of shell 930; however, lining 932 may also extend beyond the rim as shown in Figure 12, thus providing additional contact area for contact with cervix 914 as well as a readily deformable band 933 that surrounds the rim of shell 930. The rim of shell 930 should not extend into the fornices as will be discussed in greater detail hereinbelow.

Foam lining 932 is soft and pliant, and has a hardness value that is lower than that of cervix 914. In this manner, when lining 932 contacts exocervical surface 912 as cap 910 is positioned onto cervix 914, lining 932 is deformed by cervix 914 to assume a mating configuration with exocervical surface 912. Stated in another way, the outer surface 934 of lining 932 is substantially complementary with exocervical surface 912 when cap 910 is in place. In this manner a mucous discharge from uterus 916 is channeled between exocervical surface 912 and lining 932 as a thin film, and the surface tension of the mucus holds cap 910 firmly in place. To avoid absorption of mucus within foam lining 932, a smooth membrane or integral skin is provided on the outer surface 934 thereof.

If it is intended to wear cap 910 for extended periods of time, e.g., for time periods of about one month or longer, a provision is made to accommodate relatively larger volumes of uterine discharge such as menstrual flow. To this end aperture 936, covered with elastomeric web 938 is provided at about the apex of shell 930. Aperture 936 is defined by shell 930 so as to be adjacent to cervical os 924 when cap 910 is in place. Elastomeric web 938 is integral with shell 930 and together with the covered outer surface portion 940 of shell 930 provides a one-way valve means that communicates with aperture 936 and defines a channel for unidirectional flow in the direction away from cervical os 24 and through aperture 936, but not in the reverse direction. For enhanced valving action, elastomeric web 938 can be provided with protuberance or button 942 that extends into aperture 936 and seats against the inner edge surface of shell 930 that defines aperture 936. Protuberance 942 can be unitary with web 938 or can be initially separately

formed and then bonded to web 938 so as to be integral therewith. However, a flap valve utilizing a web of substantially uniform thickness as illustrated in Figure 15 is also suitable.

The above-described one-way valve means opens under increased, predetermined uterine pressure, usually at a pressure of about 10 to about 15 millimeters of mercury, and, when open, permits the passage of fluids such as mucus, menstrual flow, and the like, therethrough without disturbing the positioning of cervical cap 910 and while preventing the entry of sperm into fundus 922.

Another embodiment of the present invention is illustrated by valved cervical cap 945 shown in Figure 13. In this particular embodiment, cap 945 includes flexible outer shell 947, foam inner liner 949, and one-way valve means 951. Foam inner liner 949 comprises foam layer 952 bonded to shell 947 on one side and to relatively thin, soft and flexible liner member 955 on the other. Foam layer 952 is thicker at rim 958 of outer shell 947 and gradually tapers down in thickness as layer 952 extends deeper into shell 947. However, to accommodate some cervix contours it may be desirable to have the foam lining of substantially uniform thickness throughout or even thicker at the apex of the shell. In the embodiment illustrated in Figure 13, foam layer 952 extends from the rim of shell 947 to about aperture 953 at the apex of shell 947. Preferably, the thickest region of layer 952 is thicker than the outer shell 947.

One-way valve means 951 comprises relatively thin, elastomeric membrane 954 peripherally bonded to the outer convex surface of shell 947 along the major portion of web periphery so as to define a tapered pocket overlying aperture 953 and thus a channel terminating in discharge port 956 (Figure 14) near rim 958 of shell 947. In Figures 13 and 14 bead 957 delineates the bond between web 954 and shell 947. Discharge port 956 is spaced from aperture 953, preferably by at least one aperture diameter and, most preferably, is situated at or near rim 958 of shell 947. Most preferably discharge port 956 is situated at or no more than about 1 to about 3 millimeters away from rim 958. Elastomeric membrane or web 954 preferably is provided with unitary protuberance 959 that extends into aperture 953 and seats against edge 960 of shell 947 defining the aperture. Preferably edge 960 and the juxtaposed surface of protuberance 959 are oppositely chamfered so as to increase the area of sealing contact between protuberance 959 and edge 960.

To avoid dislodgement of the cervical cap such as cap 910 or cap 945 during momentary but relatively severe uterine displacement, it is important that the outermost edge of the cap, defined by rim 958 (Figure 13), is sufficiently elastic to remain in contact with that portion of exocervical surface that is contiguous therewith even during such momentary displacement. To this end, it is desirable that the outermost edge of the cap be feathered, i.e., beveled or tapered as shown in Figures 12 and 13. The feathered outermost edge of the cap can also be provided with a plurality of peripheral cuts to form a clipped or frilled edge.

Tactile orientation marker 962 (Figure 14) in the form of a protuberance or a discernible depression can be provided for facilitating orientation of valved cap 945 upon insertion so as to position discharge port 956 in or near the posterior fornix.

Shell 947, and thus cap 945, can be prefabricated in various standard sizes to accommodate the normally-encountered cervical configurations. For any given cervix, or range of cervices, shell 947 is dimensioned so as to have a diameter and depth sufficient to receive a major portion of the portio; however, the rim of shell 947 should terminate short of the fornices vaginae so as to guard against dislodgement of the cervical cap during activity that may cause the uterus to shift position and/or distend the normal configuration of the fornices. On the other hand, the readily deformable band of the cap inner liner that projects beyond the rim of shell 947 may extend into the fornices since any substantial deformation or distortion of the fornices will also bring about a corrresponding complementary deformation of the produced deformable band, without causing the cap to be dislodged.

Another type of valved cervical cap embodying the present invention is shown in Figure 15. In this particular embodiment the entire inner concave surface of shell 965 is lined with flexible foam liner 967 having a smooth, unitary skin 969. Aperture 971 is provided in shell 965 at the apex thereof. Elastomeric web 973 covers aperture 971 and is bonded to the outer, convex surface of shell 965 in a manner similar to that for web 954 in Figure 14, i.e., a fan-shaped web is secured over aperture 971 and is bonded to shell 965 along bead 974 so as to define a pocket or channel and a discharge port at or near the rim of the cap. Web 973 is of substantially uniform thickness.

In use, the valved cervical caps embodying the present invention are positioned on the cervix so that the valve discharge port or external opening defined by the aperture-overlying elastomeric web in conjunction with the convex outer surface of the shell is located in or near posterior fornix. Such an arrangement results in a manfiold increase in the distance that any sperm deposited in the vagina must travel in order to reach the cervical os.

Referring back to Figure 12, a portion of the increased distance for sperm travel is in vagina 918 where the environment is very inhospitable to sperm motility due to the relatively low ambient pH. Usually sperm can survive in the vaginal vault only for a time period of about one to two hours. Inasmuch as sperm can move at a velocity of about one to two millimeters per minute, the substantial increase in the distance that sperm must travel when a valved cervical cap embodying the present invention is in place alone markedly reduces the likelihood of fertilization. Sperm travel to cervical os 924 via the valve means provided in the cervical cap is possible, if at all, only in close proximity to the walls defining the valve means. In instances where the shell portion of the cervical cap, or both the shell portion and the elastomeric web bonded

thereto, are made of a thermoplastic elastomeric material, which materials exhibit an inhibitory effect on sperm motility, the likelihood of sperm reaching the cervical os 924 is further reduced.

The shell portion of the cervical cap can be made from a wide variety of flexible, non-porous materials such as natural rubber, certain types of silicone rubber that are non-porous when cured, polyurethane, thermoplastic elastomers, such as polyolefin blends, styrene/elastomer block copolymers, copolyesters, and polyurethane block copolymers. Particularly suitable are the styrene/elastomer block copolymers such as those commercially available from the Shell Chemical Company, Oak Brook, Illinois, under the designation "Kraton" and "Kraton G" and described in U.S. Patent No. 3,231,635 to Holden et al. These styrenic thermoplastic elastomers are block copolymers of polystyrene and an elastomer such as polyisoprene, polybutadiene, ethylene-propylene, or ethylene-butylene rubber. While these thermoplastic elastomers differ chemically, their morphology is similar. Blocks or domains of relatively hard thermoplastic constituents link elastomeric constituents in a network that behaves like a chemically crosslinked rubbery structure. At forming temperatures the relatively hard thermoplastic domains of the structure soften and allow the polymeric material to flow. Upon cooling, these relatively hard domains resolidify and re-establish the rubber-like, elastic structure.

Thermoplastic elastomers that exhibit a surface charge provide a further advantage for the present purposes in that the presence of such a charge on the fabricated shell and or the web tends to inhibit sperm motility and may disorient the molecular structure of cervical mucous, inhibiting sperm transit.

While thermoplastic elastomeric materials of varying hardness may be used to fabricate the shell portion of the cap, for optimum stability against dislodgement preferably the material should be harder than the cervical tissue that comes in contact therewith, yet the hardness should not be so high as to cause discomfort to the wearer or her consort. Preferably the shell material has a Shore A durometer hardness value of about 35 to about 70, and more preferably of about 45 to about 60. The shell material can be opaque, semi-transparent or transparent.

The web for the one-way valving means can be made from the same elastomeric material or from a different, non-porous elastomeric material, as the shell, such as natural rubber, silicone rubber, polyurethanes, fluorocarbon rubbers, styrene-butadiene rubbers, and the like. For ease of manufacture it is preferred to have the web of the same elastomeric material as the shell material. The web can be unitary with the shell or integrally bonded to, and thus integral with, the shell. In general, however, the web thickness is selected so as to provide a minimal valve-opening pressure, usually about 5 millimeters of mercury or less.

The sheet-like liner sheath can be made of non-porous medical grade silicone rubber, natural rubber or the like. If to be left in place, the linear sheath should be fluid impermeable, non-porous, smooth and of a wettable material which is non-irritating to cervical tissue. In any event, the cap surfaces should be such as not to harbor undesirable microorganisms.

The plugs can be made of natural rubber, medical grade silicone rubbers or the like.

For cervical cap assemblies having a wettable, non-porous permanent liner sheath defining the contact surface with the cervix uteri, elastomeric layer 36 can be custom molded using a non-wettable medical grade silicone rubber. Physiologically compatible, two-component room temperature vulcanizing or vulcanizable, non-wettable silicone rubber suitable for the present purposes, also referred to as "RTV silicone rubber", is commercially available under the trademark Silastic 382 or Silastic 502 medical grade elastomer from Dow Corning Corporation. Silastic 382 medical grade elastomer consists substantially of an opaque viscous elastomer base of polydimethylsiloxane polymer, silica filler, and stannous octoate as catalyst. Typical properties for Silastic 382 medical-grade elastomer are shown in the table below:

TABLE I
Silastic 382 medical grade elastomer
Property—Test method

Before catalyzing
- Viscosity, centipoises, 50,000

After vulcanizing for 24 hr. at 77°F
- Color, White
- Specific gravity, 1.13
- Durometer hardness, Shore A, ASTM D676, 43
- Tensile strength, psi ASTM D412, 400
- Elongation, % ASTM D412, 160
- Die B tear strength, psi ASTM D624, 25
- Brittle point, °F·ASTM D746, −100
- Shrink, linear, %
  - after 3 days at 77°F 0.4
  - after 6 days at 77°F 0.6
  - after 14 days at 77°F 0.7
- Water absorption, wt.-%, after 7 days immersion at 77°F 0.4
- Thermal conductivity, cal/cm°C sec, $0.525\times10^{-3}$
- Volume coefficient of thermal expansion/°C (0 to 100), $750\times10^{-4}$

Two-component RTV silicone rubber cures in a relatively short time and is formed by mixing the catalyst into the elastomer base to bring about vulcanization. The elastomer base includes the fluid silicone polymer mixed with filler and a material designed to act as a cross-link. Because of the necessity of mixing in a catalyst, the viscosity of the polymer used must be low enough to permit easy stirring. Also, the fillers used must not thicken too greatly. Medical-grade RTV silicone rubber vulcanized with stannous octoate shows no more tissue reaction than the other medical-grade silicones.

The process of vulcanization of the two component RTV's is as follows. The polymer used has a high degree of hydroxy end-blocking as shown by Formula I:

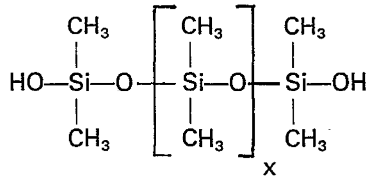

(I)

The cross-linking between these polymer chains is accomplished by a material such as propyl orthosilicate shown by Formula II:

$$Si(OCH_2-CH_2-CH_3)_4 \quad (II)$$

When the stannous octoate catalyst is stirred into a mixture composed of the compound of Formula II and a hydroxy end-blocked silicone polymer of the type shown by Formula I, the hydroxyl on the end of the polymer reacts with the alkyl group, forming an alcohol. The polymer chain then attaches to the silicate as shown below, forming the network necessary for a material to have rubbery properties is formed.

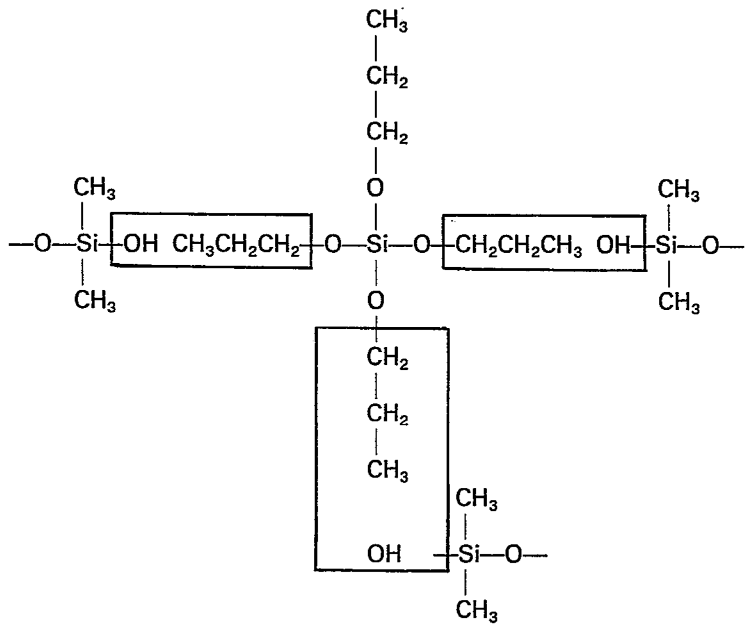

(III)

The RTV silicone rubber is formed into a moldable paste-like composition and is inserted into the space between inner surface 32 of shell 30 and liner sheath 44 during formation of cap assembly 10. The mixture is poured in the presence of a setting agent into the pocket between the concave inner surface 32 of the shell 30 and the liner sheath 44 at ambient room temperature or at a temperature at about body temperature of the woman for whom the cap is made as described above with respect to Figures 1—8 or between the concave inner surface 32 of the shell 30 and the exocervical surface 12 of the cervix uteri 14 at about body temperature as described above with respect to Figures 9 and 10. The cap assembly is then inserted against the exocervical surface 12 of the cervix uteri 14 as described above, so that the elastomeric composition will cure and set in situ to form a coherent, exocervical surface-conforming, pliable resilient layer. The preferred elastomeric composition should be molded and cured in situ in a relatively short time, such as in less than 15 minutes and preferably within three or four minutes.

Before introducing the elastomeric material 36 into the pocket between liner sheath 44 and the concave inner surface 32 of shell 30, it may be desirable in some circumstances to wash the inner surface of the shell with a solution of a strong mineral acid, such as hydrochloric acid, or to prime the surface with a dispersion made from a medical adhesive silicone, such as sold under the trademark Silastic medical adhesive silicone Type A, in dry aromatic, aliphatic or chlorinated hydrocarbon solvents. Alternatively, a mesh or screen can be connected to the inner surface of the shell to secure the elastomeric material to the cap. Furthermore, it may be desirable in some cases to pre-fill the cavity or pocket between liner sheath 44 and shell 30 and to inject the catalyst through the liner sheath into the elastomeric-forming material in the pocket at the time of use.

The moldable elastomeric material can also be formed as described in Examples 1—3 below:

Example 1

| | Parts by weight |
|---|---|
| Room temperature vulcanizing silicone rubber (Silastic RTV 502), a liquid prepolymer dimethyl silicone, about 45,000 cs | 30 |
| Silicone rubber foam (Silastic Q-3-0030), intumescent, about 5,000 cs | 30 |
| Cyclic dimethylpolysiloxane (silicone fluid), 20 cs | 40 |
| Stannous octoate (catalyst) | 2 |

The viscosity of the material, immediately after the catalyst is added, and before setting begins appreciably, is less than 6,000 centistokes.

In using the composition of Example 1, the catalyst is added immediately before the material is used. The material can be put into a flexible, liquid impervious bag and mixed.

Example 2

| | Parts by weight |
|---|---|
| Room temperature vulcanizing silicone rubber (Silastic RTV 502), a liquid prepolymer dimethyl silicone, about 45,000 cs | 50 |
| Silicone rubber foam (Silastic Q-3-0030), intumescent, about 5,000 cs | 50 |
| Cyclic dimethyl polysiloxane (silicone fluid), 20 cs | 2 |

The viscosity of the material, immediately after the catalyst is added, and before setting begins appreciably, is less than 6,000 centistokes.

Example 3

The following ranges of the silicone ingredients of Examples 1 and 2 are operative. Other proportions may be useful.

| | Parts by weight |
|---|---|
| Silastic RTV 502 | 10—40 |
| Silastic Q-3-0030 | 70—30 |
| Silicone fluid | 5—40 |

In any of Examples 1, 2 and 3, other RTV silicone rubbers and silicone fluids may be used. The "Silastic" products are commercially available from Dow Corning Corporation. Other suitable RTV silicone rubbers are commercially available from General Electric, designated RTV 11 and RTV 40, and RTV 120 and RTV 160 respectively. These latter are also curable with stannous octoate.

Another suitable silicone elastomer for the foregoing purposes is Silastic 390, available as a two-component, self-curing system under the designation "Prolastic 35" from Prolastic Co., Inc., Rochester, N.Y.

Where the liner sheath 44 is removed after curing and in those cases where it is desired to cure and mold the elastomeric layer directly in contact with the cervix, the elastomeric layer should provide a wettable, non-porous contact surface. In instances where the elastomeric layer is comprised of a silicone rubber, wettability can be achieved by combining with the silicone prepolymer a predetermined amount of hydroxyethyl-methacrylate, curing the prepolymer in the usual manner, and thereafter subjecting the obtained material to *gamma*-radiation. In lieu of the hydroxyethylmethacrylate, polymerizable vinyl-group containing monomers such as acrylic acid, alkali metal acrylates, vinyl pyrrolidone, or the like can be used.

Elastomeric foams such as silicone elastomer foam, flexible urethane foam and cross-linked polyethylene foam can also be used to form at least a portion of the elastomeric layer 36 as long as appropriate steps are taken to avoid fluid penetration therein, e.g., by using a liquid impervious, non-porous liner sheath to define the exposed or contact surface of the elastomeric layer, by forming a liquid impervious skin for the elastomeric layer, or the like.

Suitable elastomeric silicone foams can be prepared by mixing an organohydrogensiloxane, a hydroxylated organosiloxane, and a platinum catalyst in amounts to provide a ratio of silicon-bonded hydrogen atoms to silicon-bonded hydroxyl radicals of about 2.5 to about 40 and thereafter permitting the mixture to foam.

Suitable flexible urethane foams can be prepared by reacting relatively high molecular weight polyols with isocyanates.

High-resilience foams, with a sag factor (the ratio of the load needed to compress foam by 65% to the load needed to compress foam by 25%) of 2.7 and above, preferably in the range between about 3 to about 3.2 are particularly useful where foams are used to form the elastomeric layer.

High-resilience polyurethane foams are prepared by reacting an isocyanate mixture constituted by about 80 percent 80/20 tolylene diisocyanate ("80/20" refers to the ratio of the isomeric 2,4-tolylene diisocyanate to 2,6-tolylene diisocyanate) and about 20 percent polymethylenepolyphenyl isocyanate with a polyether triol having a molecular weight of about 4500 to about 6000 and made by reacting ethylene oxide with polypropylene oxide-based triols. Diol extenders such as methyldiethanol-amine, which also acts as a catalyst, can also be used in making high resilience foams.

Other materials can also be used to form the elastomeric layer.

To manufacture a cervical cap having a deformable liner, first a flexible shell of predetermined configuration (with or without a valve means, as desired) is molded or otherwise fabricated. Thereafter a male mold or plug is positioned within the shell. The mold or plug is dimensioned so as to provide an

annular space between the mold or plug face and the concave inner surface of the shell at least in the vicinity of the rim of the shell. The width of the annular space can vary; however, it is preferred that in the vicinity of the rim of the shell the annular space is at least as wide as the shell is thick. A predetermined amount of foamable material is then injected into the annular space and is permitted to foam in place, thereby forming the foam inner lining. The injected foamable material, as it rises in the annular space, structurally bonds itself to the inner shell surface and at the same time forms a relatively solid surface skin at the mold or plug face. The mold or plug face can be cooled, if desired, to suppress vaporization and/or expansion of the blowing agent contained in the foamable composition so that the foam expands at a much lower rate, if at all, at the mold surface.

For the fabrication of a cervical cap having a liner membrane over the foam layer within the shell, the assembling device shown in Figures 16 and 17 can be utilized. In particular, assembling device 980 comprises hollow movable mandrel 981 and stationary block 982 provided with cavity 983 adapted to receive distal end 984 of mandrel 981 therein. Cavity 983 is also dimensioned so as to receive therein a prefabricated, flexible cervical cap shell such as shell 985.

Mandrel 981 is surrounded by independently movable collar 986 slidably received thereon and having annular cavity 987 adapted to receive the rim portion of shell 985 as will be described in greater detail hereinbelow. Annular cavity is adjacent to mandrel 981 and faces distal end 984 thereof. Mandrel 981 further defines central, longitudinally-extending cavity 988 that communicates with a vacuum source (not shown) via passageway 989. Distal end 984 of mandrel 981 is provided with a plurality of passageways 990, 991 and 999 that are in communication with central cavity 988 and is dimensioned for entry into block cavity 983.

Situated within cavity 988 is hollow movable piston 992 having central passageway 993 in communication with a source of air under positive pressure (not shown). Through passageways 994 and 995 are provided in the distal end of piston 992. Spaced o-rings 996 and 997 are carried by piston 992, one on each side of passageways 994 and 995.

To begin fabrication of a cervical cap embodying the present invention, shell 985 is positioned in cavity 983. With piston 992 in the position shown in Figure 16, prefabricated liner membrane 998 is held on distal end 984 by means of vacuum drawn through passageway 989 and is placed within shell 985 as shown. If desired, mandrel 981 can also be an ultrasonic horn and block 982 an anvil therefor, so that liner membrane 998 can be ultrasonically bonded to shell 985 as mandrel 981 is lowered into shell 985 and presses liner membrane 998 thereagainst.

Foamable substance 900 is introduced into the space between shell 985 and liner membrane 998, and foaming initiated in any convenient manner, e.g., by heating block 982, by catalytic action, or the like. After the foaming action has subsided and the produced foam layer extends substantially to the rim of shell 985 or beyond, piston 992 is moved downwardly within cavity 988 so as to seal off vacuum passageway 989 and to line up passageway 994 with passageway 991 and passageway 995 with passageway 999 as shown in Figure 17. As a result, air under positive pressure is blown through the aligned passageways, urging the adjacent rim portion of liner membrane 998 away from mandrel 981. Thereafter collar 986 is brought down against block 982, in the process folding the aforementioned rim portion over foam layer 901 and bonding the rim portion to shell 985 and foam layer 901. Depending on the type of foamable material utilized, the adhesive properties of this material may be adequate to effect the desired bonding between liner membrane 998, foam layer 901 and shell 985. In the alternative, additional appropriate bonding agent may be applied to the surfaces that are to be bonded. If thermoplastic materials of construction are used, bonding may also be effected by heat sealing.

Next, mandrel 981 is elevated away from block 982, carrying the finished cap with it. Subsequent upward positioning of collar 986 and piston 992 with respect to distal end 984 "breaks" the vacuum in cavity 988 by opening passageways 991 and 999 to ambient atmosphere, thereby releasing the finished cap from mandrel 981.

The aforedescribed manufacturing procedure has been described with respect to the fabrication of a valve-less cervical cap in the interests of conciseness and clarity. However, it will be appreciated that the same expedient can be utilized to fabricate a valved cervical cap with but minor modifications to distal end 984 of the mandrel to accommodate the valve aperture. That is, distal end 984 is provided with an outwardly extending boss dimensioned to receive the opening in the liner membrane that corresponds to the valve aperture in the shell portion of the cap and also to enter within the valve aperture in the shell portion so as to properly orient the liner membrane and the shell with their respective apertures in registry.

The foam lining can be formed with an integral skin formed during the molding process, or a separate liner membrane can be provided. For the latter, the web materials discussed hereinabove in connection with the one-way valve means are suitable.

Although specific embodiments of the invention have been shown and described, it is to be understood that various modifications and substitutions, as well as rearrangement of parts and steps, can be made by those skilled in the art without departing from the scope of this invention as defined by the claims.

**Claims**

1. A cervical cap with a flexible shell (30) having a concave inner surface (32) of sufficient depth to receive a major portion, but not all of a cervix uteri, characterized by a form-assuming internal liner (44) covering the concave inner surface defining a wettable, matingly fitting and complementing contact surface for the exocervical surface of the cervux uteri.

2. A cervical cap with a flexible, non porous shell (30) having a concave inner surface (32), characterized by a flexible, non-porous liner sheath (44) connected to and covering the inner surface of the shell for contacting a cervix uteri; and an intermediate resilient cushion (36) positioned between the concave inner surface and the liner sheath, the liner sheath and cushion forming a liner.

3. The cervical cap in accordance with Claim 2 wherein the cushion (36) is constructed of an elastomeric material and has an exocervical-fitting contoured surface adjacent the liner sheath (44) for matingly fitting and resiliently complementing the exocervical surface of the cervix uteri.

4. The cervical cap in accordance with Claim 2 wherein the cushion (36) is a flexible foam to provide a form assuming liner deformable upon contact with the cervix uteri without substantial deformation of a contiguous portion of the cervix uteri, the inner surface (46) of the liner sheath (44) being more flexible that the shell (30).

5. The cervical cap in accordance with Claim 2 wherein the shell (330) has a tapered edge portion (342).

6. The cervical cap in accordance with Claim 2 or 3 including a one-way valve means (34) at the apex of the shell (30) for accommodating outflow of uterine discharge but preventing inflow of material into the cap.

7. The cervical cap in accordance with Claim 6 wherein the shell (947) and liner (44) define an aperture (953) at the apex of the cap (945) and wherein an elastomeric web (954) extends over the aperture and is bonded to the shell external surface defining a channel communicating with the aperture and terminating in a discharge port (960) near the rim (958) of the shell.

8. The cervical cap in accordance with Claim 2 wherein the liner sheath (44) is constructed of a wettable material.

9. A cervical cap in accordance with Claim 1 wherein the shell (30) has an aperture at its apex substantially in alignment with the cervical os when the shell is positioned in place on the cervix, and closure means (34) associated with the shell and situated over the aperture to permit outflow of uterine discharge but preventing inflow of material into the shell through the aperture.

10. A method of forming a cervical cap assembly, comprising the steps of:

providing a non-porous shell having a concave inner surface defining an aperture and one-way valving means operatively associated with the shell about the aperture for permitting egress of uterine discharges but preventing ingress of sperm therethrough;

providing aperture covering means;

providing a material which is moldable and curable at about body temperature to an elastomeric state;

covering the aperture from within said shell with the covering means;

applying the curable material to the entire concave inner surface of the shell;

positioning the shell adjacent the exocervical surface of a cervix uteri; and

molding and curing the curable material in situ adjacent the exocervical surface to form a non-porous, but wettable contact surface matingly fitting the exocervical surface.

11. A method of forming a cervical cap assembly, comprising the steps of:

providing a flexible, non-porous shell having a concave inner surface defining an aperture and one-way valving means for permitting egress of uterine discharges but preventing ingress of sperm;

providing liquid impervious covering means including a flexible non-porous, wettable liner sheath;

providing a material which is moldable and curable at about body temperature;

attaching the covering means about the valving means from within said shell;

covering the aperture with the covering means to substantially prevent the curable material from entering into the aperture;

spacing the liner sheath along the concave inner surface of the shell to form a material-receiving pocket between the concave inner surface and the liner sheath;

introducing the curable material into the pocket;

positioning the resulting assembly onto the cervic uteri; and

molding and curing said material in the pocket in situ adjacent the exocervical surface generally conform to and matingly fit said exocervical surface.

12. Apparatus for the fabrication of a cervical cap comprising a block (982) defining a cavity (983) for receiving a flexible shell (985) having a concave inner surface; an elongate hollow mandrel (981) having a distal end (984) dimensioned for entry into the cavity and defining a central cavity (988) along the longitudinal axis of the mandrel communicating with a vacuum source, said distal end defining a plurality of passageways (990, 991, 999) communicating with the central passageway for retaining a flexible, non-porous, wettable liner sheath (998) on said distal end by means of said vacuum; a piston (992) situated within the central cavity and defining a central passageway (993) communicating with a source of air under positive pressure and having a plurality of radially extending passageways (994, 995) connected thereto, the piston being movable between a first position in which said air under positive pressure is prevented

from leaving said radial passageways and said vacuum is applied to said passageways in the distal end of the mandrel and a second position in which said vacuum is blocked off from said distal end passageways and said air under pressure blows outwardly through some of said passageways (991) in the mandrel, in order to urge a rim portion of the liner sheath away from the mandrel; and a collar (986) slidably received on the outside of the mandrel and movable into a position in which, in use, said rim portion of the liner is folded over a mouldable and curable material (900) and into contact with said flexible shell so as to form a pocket for said mouldable and curable material between said liner sheath and said shell.

**Patentansprüche**

1. Cervix-Kappe oder Pessar mit einer flexiblen Schale (30) mit einer konkaven Innenfläche (32) ausreichender Tiefe zur Aufnahme eines größeren Teiles, jedoch nicht des ganzen Gebärmutterhalses, dadurch gekennzeichnet, daß die konkave Innenfläche der Schale eine formannehmende, innere Auskleidung (44) bedeckt, welche eine benetzbare, direkt passende und ergänzende Kontaktfläche für die Außenfläche des Gebärmutterhalses bildet.

2. Cervix-Kappe oder Pessar mit einer flexiblen, nicht porösen Schale (30) mit einer konkaven Innenfläche (32), dadurch gekennzeichnet, daß eine flexible, nicht poröse Auskleidungshülle (44) an der inneren Oberfläche der Schale befestigt ist und diese bedeckt, um mit dem Gebärmutterhals in Berührung zu kommen, und daß ein zwischenliegendes federndes Kissen (36) zwischen der konkaven Innenfläche und der Auskleidungshülle vorgesehen ist, wobei die Auskleidungshülle und das Kissen eine Auskleidung bilden.

3. Pessar nach Anspruch 2, dadurch gekennzeichnet, daß das Kissen (36) aus einem elastomeren Material hergestellt ist und eine auf die Außenfläche des Gebärmutterhalses passende konturierte Oberfläche im Bereich der Auskleidungshülle (44) aufweist, um passend und federnd ergänzend der außen liegenden Oberfläche des Gebärmutterhalses zu entsprechen.

4. Pessar nach Anspruch 2, dadurch gekennzeichnet, daß das Kissen (36) aus einem flexiblen Schaum besteht, um eine die Form annehmende Auskleidung zu bilden, welche bei Berührung mit dem Gebärmutterhals verformbar ist, ohne daß es zu einer wesentlichen Verformung des berührten Teils des Gebärmutterhalses kommt, wobei die Innenfläche (46) der Auskleidungshülle (44) flexibler ist als die Schale (30).

5. Pessar nach Anspruch 2, dadurch gekennzeichnet, daß die Schale (330) einen abgeschrägten Randbereich (342) aufweist.

6. Pessar nach Anspruch 2 oder 3, dadurch gekennzeichnet, daß im Grund der Schale (30) eine Einwege-Ventileinrichtung (34) vorgesehen ist, um Gebärmutterausscheidung Rechnung zu tragen, welche Ventileinrichtung jedoch das Hineinfließen von Material in die Kappe verhindert.

7. Pessar nach Anspruch 6, dadurch gekennzeichnet, daß die Schale (947) und die Auskleidung (44) eine Öffnung (953) am Grund des Pessars (945) aufweisen, wobei eine elastomere Membrane (954) sich über die Öffnung erstreckt und mit der Außenfläche der Schale derart verbunden ist, daß die Membran einen mit der Öffnung in Verbindung stehenden Kanal bildet, welcher in einer Auslaßöffnung (960) nahe des Randes (958) der Schale endet.

8. Pessar nach Anspruch 2, dadurch gekennzeichnet, daß die Auskleidungshülle (44) aus einem benetzbaren Material hergestellt ist.

9. Pessar nach Anspruch 1, dadurch gekennzeichnet, daß die Schale (30) an ihrem Grund eine Öffnung aufweist, die im wesentlichen mit dem Muttermund ausgerichtet ist, wenn die Schale in ihrem Platz am Gebärmutterhals angeordnet ist, sowie eine Verschlußeinrichtung (34), die der Schale zugeordnet und über der Öffnung angeordnet ist, um das Ausfließen von Gebärmutterausscheidungen zu erlauben, das Einfließen von Material in die Schale durch die Öffnung jedoch zu verhindern.

10. Verfahren zum Herstellen einer Pessareinrichtung, gekennzeichnet, durch folgende Verfahrensschritte:

Bereitstellen einer nicht porösen Schale mit einer konkaven Innenfläche, welche eine Öffnung aufweist und eine Einwege-Ventileinrichtung, die betriebsmäßig mit der Schale über der Öffnung verbunden ist, um das Austreten von Gebärmutterausscheidungen zu erlauben, jedoch das Eintreten von Sperma durch die Öffnung zu verhindern;

Bereitstellung von einer Einrichtung zum Abdecken der Öffnung;

Liefern eines Materials, welches formbar ist und bei etwa Körpertemperatur in einen elastomeren Zustand aushärtbar ist;

Abdecken der Öffnung vom Inneren der Schale mit der Abdeckeinrichtung;

Auftragen des aushärtbaren Materials auf die konkave Innenfläche der Schale;

Positionierung der Schale im Bereich der außen liegenden Oberfläche eines Gebärmutterhalses; und

Formen und Aushärten des aushärtbaren Materials in situ an der außen liegenden Oberfläche des Gebärmutterhalses zum Ausbilden einer nicht porösen, jedoch benetzbaren Kontaktfläche, welche direkt auf die außen liegende Fläche des Gebärmutterhalses paßt.

11. Verfahren zum Herstellen eines Pessars, gekennzeichnet, durch folgende Verfahrensschritte:

Bereitstellen einer flexiblen, nicht porösen Schale mit einer konkaven Innenfläche, die eine Öffnung

aufweist und eine Einwege-Ventileinrichtung, welche das Austreten von Gebärmutterausscheidungen erlaubt, jedoch das Eintreten von Sperma verhindert;

Bereitstellen einer flüssigkeitsundurchlässigen Abdeckeinrichtung einschließlich einer flexiblen, nicht porösen, benetzbaren Auskleidungshülle;

Bereitstellen eines Materials, welches formbar und bei etwa Körpertemperatur aushärtbar ist;

Anbringen der Abdeckeinrichtung über der Ventileinrichtung vom Inneren der Schale her;

Abdecken der Öffnung mit der Abdeckeinrichtung, um zu verhindern, daß das aushärtbare Material in die Öffnung eindringt;

im Abstand halten der Auskleidungshülle längs der konkaven Innenfläche der Schale zur Ausbildung einer materialaufnehmenden Tasche zwischen der konkaven Innenfläche und der Auskleidungshülle;

Einführen des aushärtbaren Materials in die Tasche;

Positionieren der daraus hergestellten Anordnung auf einen Gebärmutterhals; und

Formen und Aushärten des Materials in der Tasche in situ an der Außen liegenden Oberfläche des Gebärmutterhalses, um allgemein mit der außen liegenden Oberfläche des Gebärmutterhalses übereinzustimmen und direkt du passen.

12. Vorrichtung zur Herstellung eines Pessars, gekennzeichnet, durch einen Block (982) mit einer Höhlung (983) zur Aufnahme einer flexiblen Schale (985) mit einer konkaven inneren Oberfläche, einen langgestreckten, hohlen Dorn (981) mit einem entfernt liegenden Ende (984), welches derart bemessen ist, daß es in die Höhlung eindringen kann, und welches eine zentrale Höhlung (988) längs der Längsachse des Dornes aufweist, die mit einer Vakkuumquelle in Verbindung steht, wobei das entfernt liegende Ende eine Mehrzahl von Kanälen (990, 991, 999) definiert, die mit dem zentralen Kanal in Verbindung stehen, um mittels des Vakuums eine flexible, nicht-poröse, benetzbare Auskleidungshülle (998) auf dem entfernt liegenden Ende zu halten, einen in der zentralen Höhlung angeordneten Kolben, der einen zentralen Durchgang (993) definiert, welcher mit einer Druckluftquelle in Verbindung steht und welcher eine Mehrzahl von hiermit verbundenen und sich radial erstreckenden Kanälen (994, 995) aufweist, wobei der Kolben zwischen einer ersten Position, in welcher verhindert wird, daß die Druckluft die radialen Kanäle verläßt, und in welcher an die Kanäle in den entfernt liegenden Enden des Dorns das Vakuum angelegt wird, und einer zweiten Position bewegbar ist, in der das Vakuum von den Kanälen in dem entfernt liegenden Ende abgeschaltet ist und die Druckluft durch einige der Kanäle (991) im Dorn nach außen bläst, um einen Randbereich der Auskleidungshülle weg vom Dorn zu drücken, sowie durch einen Kragen (986), der auf der Außenseite des Dorns gleitbar aufgenommen ist und in eine Position bewegbar ist, in der beim Gebrauch der Randbereich der Auskleidung über ein formbares und härtbares Material (900) und in Kontakt mit der flexiblen Schale gefaltet wird, so daß für das formbare und härtbare Material zwischen der Auskleidungshülle und der Schale eine Tasche ausgebildet wird.

**Revendications**

1. Diaphragm comportant une coque flexible (30) présentant une surface intérieure concave (32) de profondeur suffisante pour recevoir une partie importante, mais non la totalité, d'un col utérin, caractérisé par un revêtement intérieure conformable (44) recouvrant la surface intérieure concave et définissant une surface mouillable de contact s'ajustant étroitement et de façon complémentaire sur la surface exocervicale du col utérin.

2. Diaphragme comportant une coque flexible non poreuse (30) présentant une surface intérieure concave (32), caractérisé par une gaine de revêtement flexible, non poreuse (44) reliée à et recouvrant la surface intérieure de la coque pour être en contact avec un col utérin; et un coussinet élastique intermédiaire (36) placé entre la surface intérieure concave et la gaine de revêtement, la gaine de revêtement et le coussinet formant un revêtement.

3. Diaphragme selon la revendication 2, dans lequel le coussinet (36) est réalisé en une matière élastomérique et présente une surface profilée s'ajustant à la surface exocervicale, adjacente à la gaine de revêtement (44) afin de s'ajuster étroitement et élastiquement, de façon complémentaire, sur la surface exocervicale du col utérin.

4. Diaphragm selon la revendication 2, dans lequel le coussinet (36) est en mousse flexible de façon à prendre une forme permettant au revêtement d'être déformé lors de l'entrée en contact avec le col utérin, sans déformation important d'une partie contiguë du col utérin, la surface intérieure (46) de la gaine de revêtement (44) étant plus flexible que la coque (30).

5. Diaphragme selon la revendication 2, dans lequel la coque (330) comporte une partie de bord effilée (342).

6. Diaphragme selon la revendication 2 ou 3, comprenant une valve unidirectionnelle (34) au sommet de la coque (30) pour permettre les écoulements utérins mais empécher l'entrée de matière dans le diaphragme.

7. Diaphragme selon la revendication 6, dans lequel la coque (947) et le revêtement (44) définissent une ouverture (953) située au sommet du diaphragme (945) et dans lequel une voile élastomérique (954) s'étend sur l'ouverture et est lié à la surface extérieure de la coque définissant un canal communiquant avec l'ouverture et se terminant dans un orifice (960) d'écoulement proche du bord (958) de la coque.

8. Diaphragme selon la revendication 2, dans lequel la gaine de revêtement (44) est réalisée en matière mouillable.

9. Diaphragme selon la revendication 1, dans lequel la coque (30) présente à son sommet une ouverture sensiblement en alignement avec l'orifice cervical lorsque la coque est placée en position sur le col, et des moyens de fermeture (34) associés à la coque et situés au-dessus de l'ouverture pour permettre le passage des écoulements utérins, mais empêcher l'entrée de matière dans la coque par l'ouverture.

10. Procédé pour former un ensemble à diaphragme, comprenant les étapes qui consistent:

à utiliser une coque non poreuse présentant une surface intérieure concave définissant une ouverture et une valve unidirectionnelle associée fonctionellement à la coque, autour de l'ouverture pour permettre la sortie d'écoulements utérins, mais empêcher l'entrée du sperme;

à utiliser des moyens de recouvrement de l'ouverture;

à utiliser une matière qui peut être moulée et durcie sensiblement à la température du corps pour prendre un état élastomérique;

à recouvrir l'ouverture à l'aide des moyens de recouvrement à partir de l'intérieur de ladite coque;

à appliquer la matière durcissable sur la totalité de la surface intérieure concave de la coque;

à placer la coque à proximité immédiate de la surface exocervicale d'un col utérin; et

à mouler et faire durcir la matière durcissable in situ, à proximité immédiate de la surface exocervicale pour former une surface de contact non poreuse, mais moullable, s'ajustant étroitement sur la surface exocervicale.

11. Procédé pour former un ensemble à diaphragme, comprenant les étapes qui consistent:

à utiliser une coque flexible, non poreuse, présentant une surface intérieure concave définissant une ouverture, et une valve unidirectionnelle pour permettre la sortie d'écoulement utérins, mais empêcher l'entrée du sperme;

à utiliser des moyens de recouvrement, imperméables aux liquides, comprenant une gaine de revêtement flexible, mouillable, non poreuse;

à utiliser une matière qui peut être moulée et durcie sensiblement à la température du corps;

à fixer les moyens de recouvrement autour de la valve, depuis l'intérieure de ladite coque;

à recouvrir l'ouverture avec les moyens de recouvrement pour empêcher sensiblement la matière durcissable d'entrer dans l'ouverture;

à espacer la gaine de revêtement le long de la surface intérieure concave de la coque pour former une poche de réception de matière entre la surface intérieure concave et la gaine de revêtement;

à introduire la matière durcissable dans la poche;

à positionner l'ensemble résultant sur le col utérin; et

à mouler et faire durci ladite matière dans la poche, in situ, à proximité immédiate de la surface exocervicale, pour qu'elle épouse globalement ladite surface exocervicale et s'y adjuste étroitement.

12. Appareil de fabrication d'un diaphragme comprenant un bloc (982) définissant une cavité (983) destinée à recevoir une coque flexible (985) présentant une surface intérieure concave; un mandrin allongé creux (981) présentant une extrémité distale (984) dimensionnée pour pénétrer dans la cavité et définissant une cavité centrale (988) de long de l'axe longitudinal du mandrin, en communication avec une source de vide, ladite extrémité distale définissant plusieurs canaux (990, 991, 999) communiquant avec le canal central afin de retenir une gaine de revêtement flexible, mouillable, non poreuse (998) sur ladite extrémité distale au moyen dudit vide; un piston (992) situé à l'intérieur de la cavité centrale et définissant un canal central (993) communiquant avec une source d'air sous pression positive et présentant plusieurs canaux (994, 995) s'étendant radialement et reliés à la source d'air, le piston étant mobile entre une première position dans laquelle ledit air sous pression positive ne peut sortir desdits canaux radiaux et ledit vide est appliqué auxdits canaux de l'extrémité distale du mandrin, et une seconde position dans laquelle ledit vide est isolé des canaux de ladite extrémité distale et ledit air sous pression positive s'écoule vers l'extérieur dans certains desdits canaux (991) du mandrin, afin de repousser une partie du bord de la gaine de revêtement et de l'éloigner du mandrin; et une bague (986) montée de façon à pouvoir coulisser sur l'extérieur du mandrin et mobile jusqu'à une position dans laquelle, pendant l'utilisation, ladite partie de bord du revêtement est repliée sur une matière moulable et durcissable (900) et amenée en contact avec ladite coque flexible afin de former une poche pour ladite matière moulable et durcissable entre ladite gaine de revêtement et ladite coque.

Fig.1.

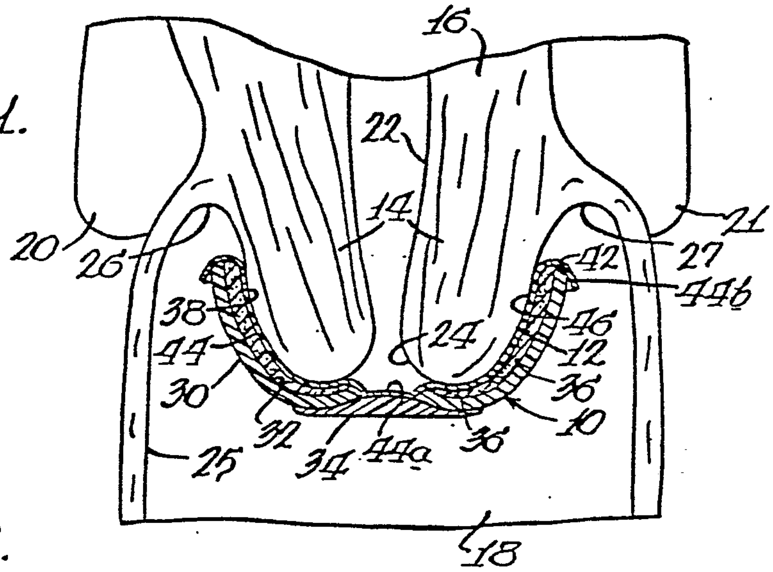


Fig.2.

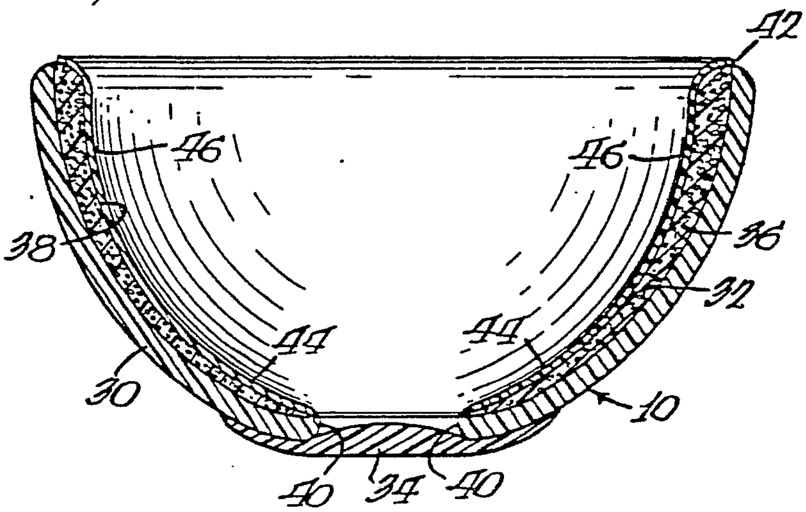


Fig.3.

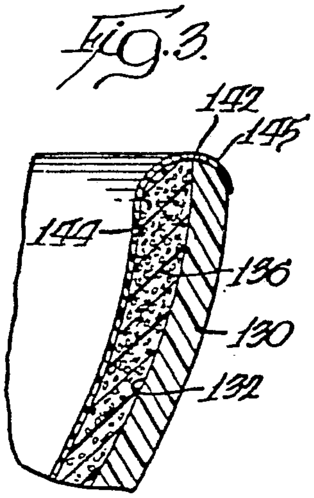


Fig.4.

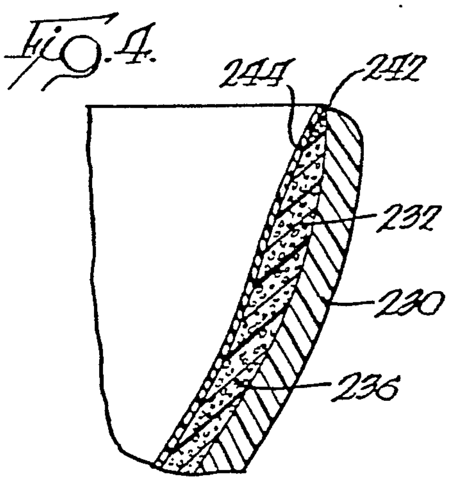


Fig.5.

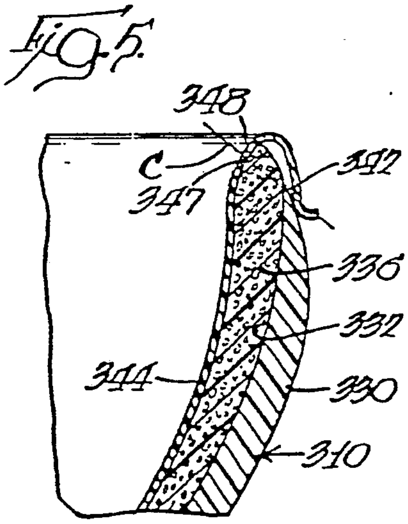

Fig.6.

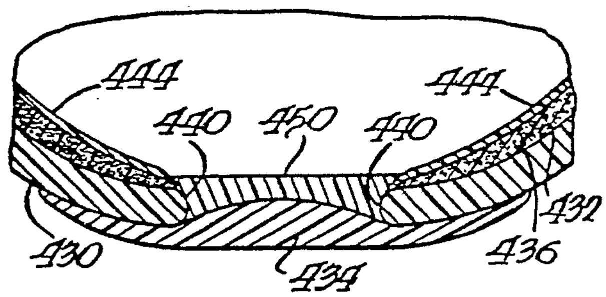


Fig.7.

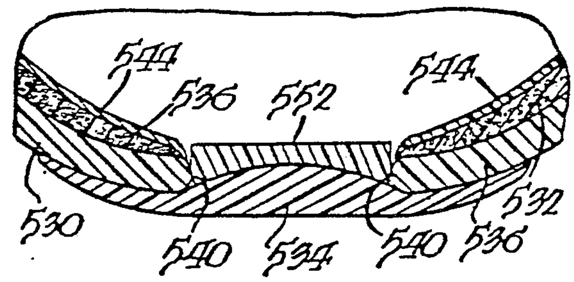


Fig.8.

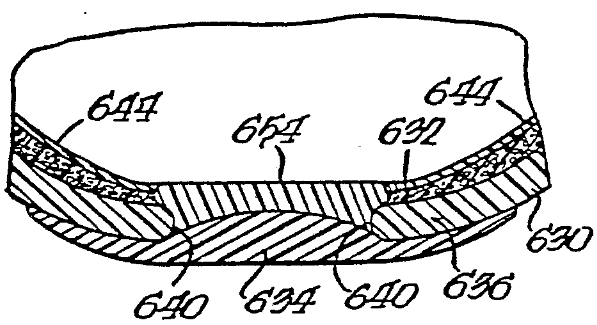


Fig.9.

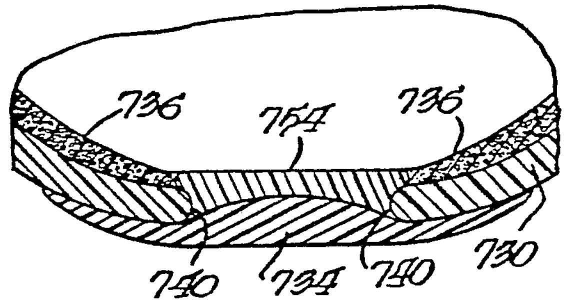


Fig.10.

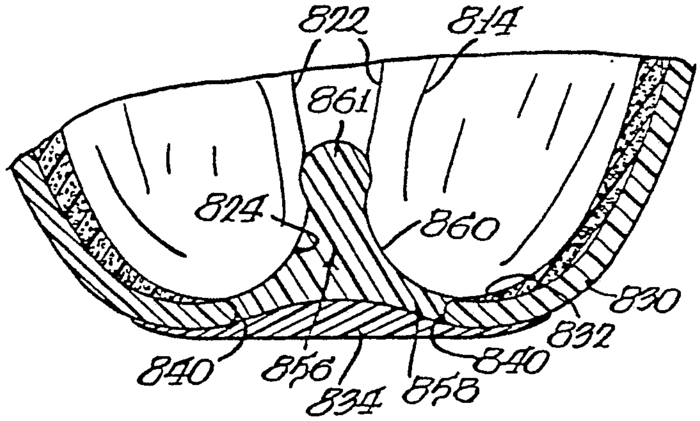


Fig.11.

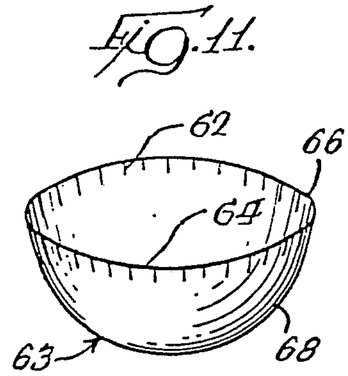

Fig. 12.

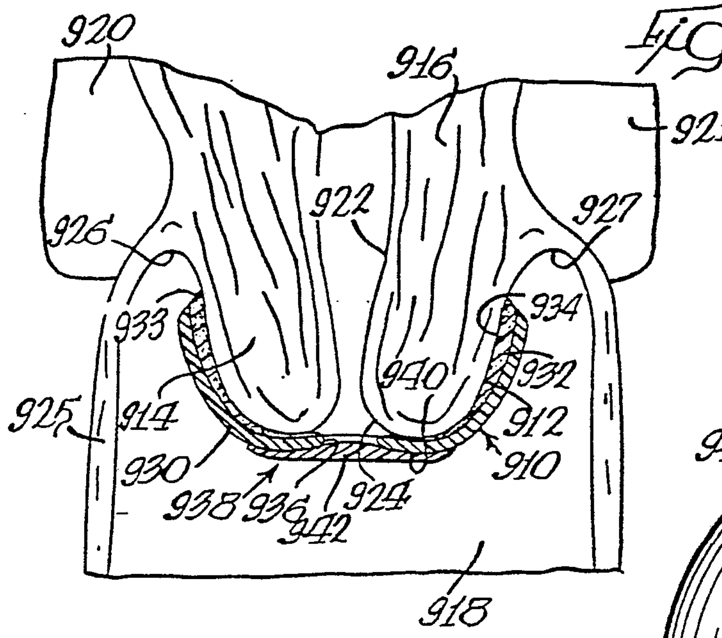


Fig. 14.

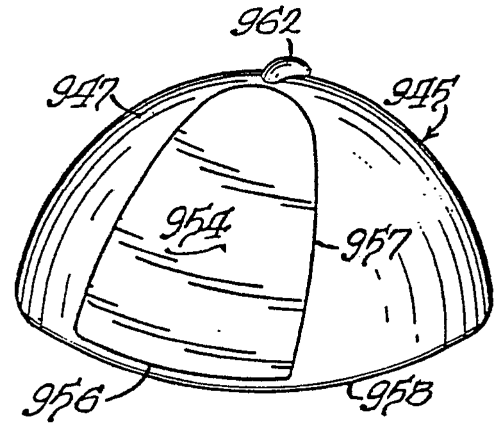


Fig. 13.

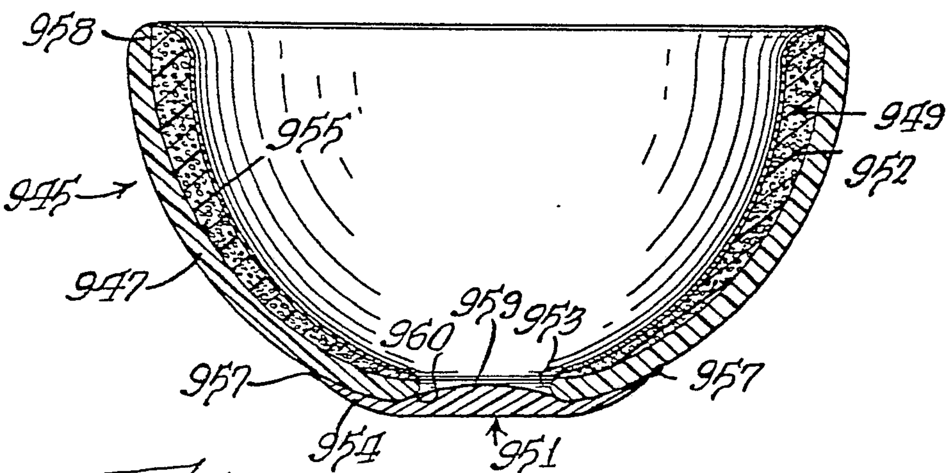


Fig. 15.

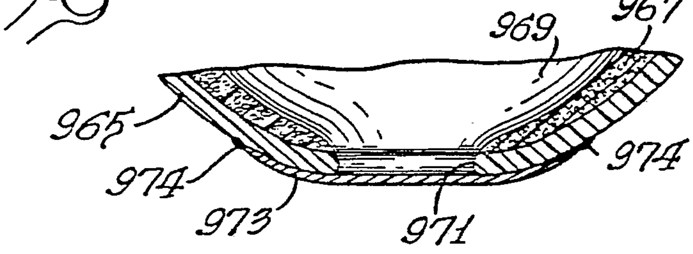

Fig.16.

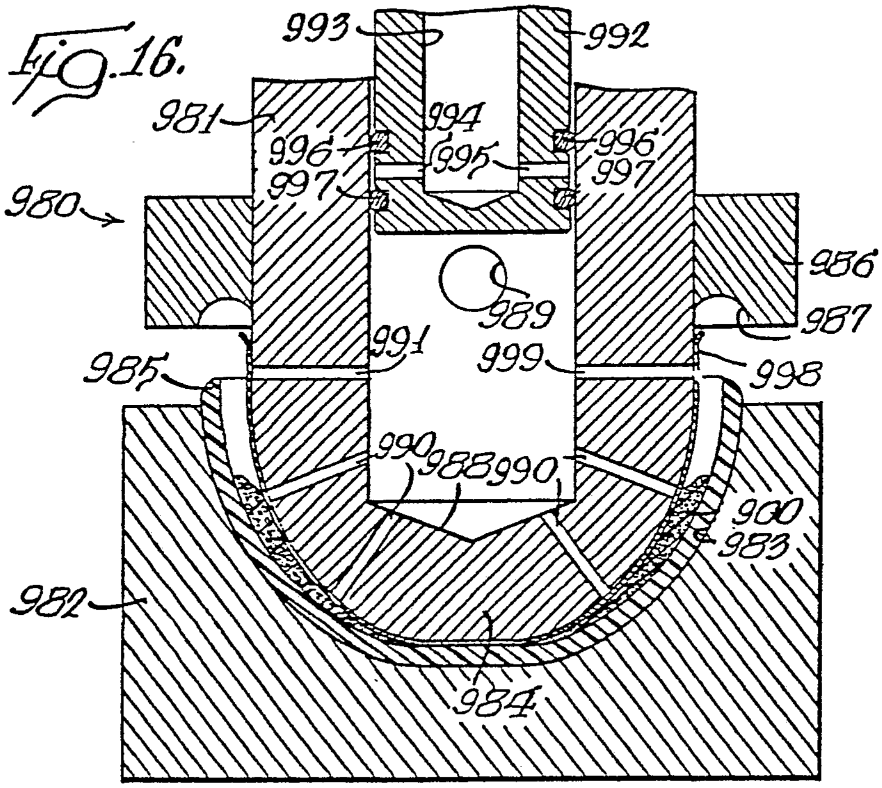
993
992
981
994
996
996
995
997
997
980
986
989
987
985
991
999
998
990
988
990
900
983
982
984

Fig.17.

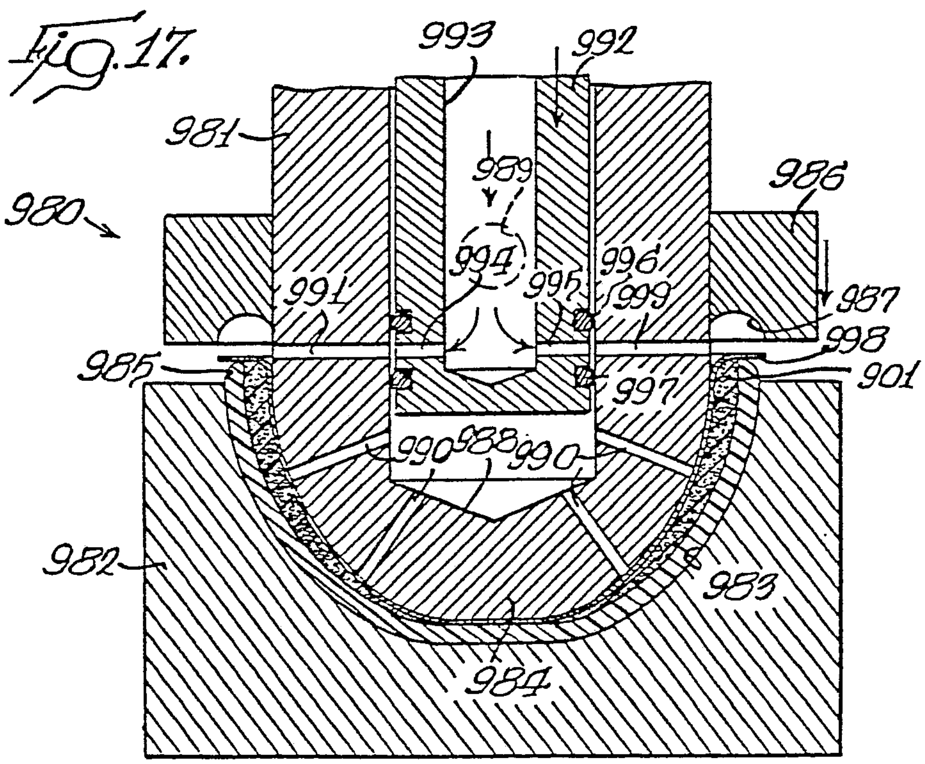
993
992
981
989
986
980
994
996
991
995
999
987
998
985
901
997
990
988
990
982
983
984